# EUROPEAN PATENT APPLICATION

(11) **EP 4 317 421 A1**
(43) Date of publication of application: **07.02.2024**
(21) Application number: 22778348.7
(22) Date of filing: 27.01.2022
(51) Int. Cl.: C12N 5/078, A61K 39/39

(54) **IMMUNE PREPARATION, COMPOSITION COMPRISING IMMUNE PREPARATION AND USE THEREOF, AND PREPARATION METHOD**

(30) Priority: 29.03.2021 CN 202110336240
(71) Applicant: Huazhong University of Science and Technology, Wuhan City Hubei 430074 (CN)
(72) Inventor: HUANG, Bo, Wuhan, Hubei 430074 (CN); TANG, Ke, Wuhan, Hubei 430074 (CN); MA, Jingwei, Wuhan, Hubei 430074 (CN); ZHANG, Huafeng, Wuhan, Hubei 430074 (CN)
(74) Representative: Perani & Partners S.p.A.
(86) International application number: PCT/CN2022/074173
(87) International publication number: WO 2022/206160

(57) **Abstract**

The present disclosure relates to the technical field of medicines, and particularly relates to an immune preparation, a composition comprising the immune preparation and a use thereof, and a preparation method, an immune adjuvant provided by the present disclosure is an erythrocyte-derived vesicle, and the erythrocyte-derived vesicle has an erythrocyte content and a biofilm structured wrapped outside the erythrocyte content as a whole, and the two are matched with each other, so that the erythrocyte-derived vesicle serves as an endogenous substance of the body to activate an immune system of the body and effectively enhance a cellular immune response. Moreover, in this process, no toxic or side effect is caused to the body, and the method has the advantages of high biocompatibility, safe raw material source and simple preparation process.

## Description

### TECHNICAL FIELD

The present disclosure belongs to the technical field of medicines, and specifically relates to an immune preparation, a composition comprising the immune preparation, and use and a preparation method thereof.

### BACKGROUND

Subject to various environmental factors, the incidence of human neoplastic diseases is increasing year by year. In addition, acute and chronic microbial infections still jeopardize human health. Infections and tumors are closely related to body's immune surveillance. Once immune escape occurs, the body's health will be severely endangered. It is for this reason that immunotherapy is considered as an effective biological therapy. Due to the immunosuppression from the tumor microenvironment and the constant variation and evolution of pathogenic microorganisms, however, immunotherapy often fails to achieve the desired effects. How to effectively activate the body's immune response and improve the response rate to antigens is a major issue that needs to be solved urgently in the field of immunotherapy.

Acquired immune response relies on effective presentation of an antigen by the antigen-presenting cell to form a major histocompatibility complex (MHC) - antigen complex, which further activates specific T cells under the joint action of costimulatory molecules and cytokines to thereby generate a specific T cellular immune response against the antigen. The effective presentation of antigen, the activation by costimulatory molecules, and the presence of cytokines (such as IL-2 and IL-12) are three major factors essential for the generation of T cell-specific immune responses.

By adding cytokines or immune adjuvants, it is possible to facilitate the presentation of antigens and the expression of costimulatory molecules, and then activate the acquired immune response. Of these, immune adjuvants have gained rapid development in immunotherapy. In addition to conventional aluminum adjuvants, oil-in-water emulsion MF59 and the like, agonists for Toll-like receptors (TLRs) such as cytosine phosphoguanosine oligodexynucleotide (CpG-ODN), polyinsinic-polycytidylic acid (Poly I:C), and lipopolysaccharide (LPS) have been found to have good immune activation effects. Despite the rapid development of immunology, what are currently applied in the clinical stage are still the conventional adjuvants such as aluminum adjuvants and oil-in-water emulsion MF59 as a result of concerns about the side effects of immune adjuvants. Although the aluminum adjuvants and oil-in-water emulsion MF59 may augment the body's humoral immunity, it is usually difficult for them to induce the body's cellular immunity, and their immunotherapy effects on tumors are not desirable.

At present, some synthetic carriers are also used for encapsulating antigens and adjuvants and then releasing them selectively at tumor sites, such as PLGA (poly(lactaldehyde -co-glycolic acid)), liposomes, and PEG-PE (polyethylene glycol-phosphatidylethanolamine copolymer). However, synthetic nanomaterials as exogenous substances inherently have toxic and side effects on organisms and their long-term biological toxicity cannot be evaluated yet. Besides, most of the synthetic nanoparticles are composed of complex biological or chemical composite materials, causing the carrier particles to be costly, which is not conducive to large-scale clinical promotion.

Chinese patent literature CN109718380A discloses a tumor antigen-presenting system prepared from erythrocyte membranes. The tumor antigen-presenting system is fused with tumor cell membrane under ultrasound and extruded to form nanoplasmic membrane vesicles with a structure of nexus, and the nanoplasmic membrane vesicles are effective in activating the immune system. Although it takes the bio-originated erythrocyte membrane as the tumor antigen-presenting system, there is a need to rupture the erythrocytes and collect cell membranes, and the erythrocyte membranes and the tumor cell membrane are required to be fused to achieve the anti-tumor effect, which is faced with problems of complicated steps and high cost for preparation.

Therefore, developing a novel adjuvant with a good immune activation effect, low toxic and side effects, and easiness of preparation and promotion is a major issue that needs to be settled urgently in the field of immune adjuvants.

### SUMMARY

### Technical Problem

In view of the problems existing in the prior art, for example, immune adjuvants with good biocompatibility need to work jointly with tumor cell membranes to exert immune activation effects, which have the drawbacks of a complicated preparation process and high preparation cost, the present disclosure provides an immune adjuvant, which is an erythrocyte-derived vesicle that is capable of effectively activating the expression of costimulatory factors of an antigen-presenting cell, thereby promoting formation of the body's specific immune responses such as resistance to tumors and microbial infections, and has the advantages of easy access to raw materials, safe sources, high biocompatibility, and simple preparation process.

### Solution to Problem

The present disclosure provides an immune adjuvant, wherein the immune adjuvant is an erythrocyte-derived vesicle, and the vesicle comprises an erythrocyte content and a biomembrane structure encapsulating the erythrocyte content.

In some embodiments, the immune adjuvant according to the present disclosure, wherein the vesicle is an extracellular vesicle released by an erythrocyte; optionally, the erythrocyte is a non-induced or apoptosis-induced erythrocyte.

In some embodiments, the immune adjuvant according to the present disclosure, wherein the vesicle has a particle size of 50 to 500 nm; preferably, the vesicle has a particle size of 100 to 500 nm.

In some embodiments, the immune adjuvant according to the present disclosure, wherein the immune adjuvant further comprises one or more pharmaceutically acceptable carriers; optionally, the pharmaceutically acceptable carriers comprise one or a combination of two or more of a solvent, a solubilizer, a cosolvent, an emulsifier, a corrigent, an odor-masking agent, a colorant, a binder, a disintegrant, a filler, a lubricant, a wetting agent, an osmotic pressure regulator, a pH regulator, a stabilizer, a surfactant, and a preservative.

In some embodiments, the immune adjuvant according to the present disclosure, wherein the immune adjuvant is a tablet, a capsule, an injection, a spray, a granule, a pulvis, a suppository, a pill, a cream, a paste, a gel, a powder, an oral solution, an inhalant, a suspension, or a dry suspension; preferably, the immune adjuvant is an injection.

The present disclosure further provides a composition, comprising an immune adjuvant according to the present disclosure.

In some embodiments, the composition according to the present disclosure, wherein the composition further comprises an immunotherapeutic agent; preferably, the immunotherapeutic agent is at least one selected from the group consisting of an antigen, an antigen-presenting cell, and an immunomodulator; more preferably, the antigen-presenting cell is a dendritic cell.

The present disclosure further provides use of an immune adjuvant according to the present disclosure or a composition according to the present disclosure in at least one of (a) to (c) as follows:
(a) preparing a medicament for inducing an immune response or activating an antigen-presenting cell;
(b) preparing a medicament for preventing or treating a tumor; and
(c) preparing a medicament for preventing or treating a microbial infection;
   preferably, the antigen-presenting cell is a dendritic cell.

The present disclosure further provides a method for preparing an immune adjuvant according to the present disclosure, comprising the following steps:
culturing step: culturing an erythrocyte that secretes a vesicle into a culture environment; and
collection step: collecting the vesicle in the culture environment, which is the immune adjuvant;
optionally, the culturing step further comprises subjecting the erythrocyte to apoptosis-inducing treatment, such that an apoptosis-induced erythrocyte secretes the vesicle into the culture environment;
optionally, the apoptosis-inducing treatment comprises irradiating the erythrocyte with ultraviolet light.

In some embodiments, the method according to present disclosure, wherein the collection step comprises subjecting the erythrocyte in the culture environment to density gradient centrifugation and collecting the vesicle after centrifugation; preferably, the density gradient centrifugation is carried out under the condition of 1000 to 50000 g.

### Advantageous Effects of the Invention

In some embodiments, the immune adjuvant provided in the present disclosure is an erythrocyte-derived vesicle. The erythrocyte-derived vesicles exhibit a better effect of promoting the body's immune response as compared to activation by an antigen alone or to other immune adjuvants. In the meantime, the erythrocyte-derived vesicles are highly safe and have the advantages of abundant source and easiness of preparation and access.

In some embodiments, the erythrocyte-derived vesicle is an endogenous substance of the body. The body itself has specific biodegradation pathways. Compared with other adjuvants, it has high biocompatibility and effectively avoids autoimmune diseases.

In some embodiments, the erythrocytes as the source of vesicles are derived from blood, and may be obtained by collecting directly from body's blood without the need for large-scale cell culture, which is low in cost and simple in operation.

In some embodiments, without being limited to the degree of binding to antigens, the erythrocyte-derived vesicles are capable of effectively activating the antigen-presenting cells, in particular the dendritic cells, can target the vast majority of tumors and infections, and have good universality. The immune adjuvants of the present disclosure expand the treatment type and scope of diseases, reduce the cost and risks, and make the operations simpler.

### BRIEF DESCRIPTION OF THE DRAWINGS

FIG. 1 shows the particle size and morphology of erythrocyte-derived vesicles.
FIG. 2 shows the yield of erythrocyte-derived vesicles.
FIG. 3 shows an effect of erythrocyte-derived vesicles on expression of costimulatory molecules of dendritic cells.
FIG. 4 shows an effect of erythrocyte-derived vesicles on expression of cytokines of dendritic cells.
FIG. 5 shows an effect of erythrocyte-derived vesicles and tumor lysates on activation of dendritic cells.
FIG. 6 shows activation of the body's dendritic cells by erythrocyte-derived vesicles *in vivo.*
FIG. 7 shows comparisons between activation situations of the body's dendritic cells by erythrocyte-derived vesicles and by other adjuvants.
FIG. 8 shows comparisons between activation situations of the body's specific T cells by erythrocyte-derived vesicles and by other adjuvants.
FIG. 9 shows a prophylactic effect of erythrocyte-derived vesicles as an immune adjuvant on tumor.
FIG. 10 shows a therapeutic effect of erythrocyte-derived vesicles as an immune adjuvant in combination with dendritic cells on tumor.
FIG. 11 shows a prophylactic effect of erythrocyte-derived vesicles as an immune adjuvant on *Listeria monocytogenes.*
FIG. 12 shows an effect of erythrocyte-derived vesicles as an immune adjuvant in combination with dendritic cells on survival of mice infected with *Listeria monocytogenes.*
FIG. 13 shows an influence of inoculation with erythrocyte-derived vesicles on mouse body weight.

### DETAILED DESCRIPTION

### Definitions

When used in combination with the term "including" in the claims and/or specification, the word "a" or "an" may refer to "one", or refer to "one or more", "at least one", and "one or more than one".

As used in the claims and specification, the term "including", "having", "comprising", or "containing" is intended to be inclusive or open-ended, and does not exclude additional or unrecited elements or methods and steps.

Throughout the application document, the term "about" means that a value includes the standard deviation of error caused by the device or method used to measure the value.

It is applicable to the content disclosed herein that the term "or" is defined only as alternatives and "and/or", but the term "or" used herein refers to "and/or" unless otherwise expressly stated to be only alternatives or mutual exclusion between alternatives.

The term "vesicle" used herein refers to a relatively small saccular vesicle structure and may be present intracellularly or extracellularly. In some embodiments, the vesicles of the present disclosure are extracellular vesicles released by cells into the extracellular environment. In some embodiments, the vesicles of the present disclosure are Erythrocyte Microparticles (EMPs). In the present disclosure, the terms "erythrocyte-derived vesicles", "erythrocyte-derived microparticles", "erythrocyte vesicles", "erythrocyte microparticles", and "EMP" may be used interchangeably.

The term "extracellular vesicles" (EVs) used herein refers to nanovesicles with a lipid bilayer structure that are secreted by cells. In some embodiments, the erythrocyte-derived vesicles of the present disclosure are erythrocyte-derived extracellular vesicles having an erythrocyte content and a biomembrane structure encapsulating the erythrocyte content. In some embodiments, the erythrocyte content includes bioactive substances released by erythrocytes, such as nucleic acids, proteins, and lipids. In some embodiments, the biomembrane structure encapsulating the erythrocyte content is a lipid bilayer structure. The lipid bilayer forms a saccular vesicle with a closed lumen. The erythrocyte content is present in the closed lumen formed by the lipid bilayer.

The term "microorganism" used herein refers to microorganisms (also known as pathogens) that can invade the human bodies or animal bodies and cause infections or even infectious diseases. In some embodiments, the microorganisms in the present disclosure include, but are not limited to, prions, parasites (protozoa, helminths, and medical insects), fungi, bacteria, spirochetes, mycoplasma, rickettsia, chlamydia, and viruses.

The term "antigen-presenting cell" (APC) used herein refers to a class of immune cells capable of uptaking and presenting antigens and presenting the treated antigens to T cells. In some embodiments, the antigen-presenting cell includes mononeuclear phagocytes, dendritic cells, B cells, Langerhans cells, and virus-infected target cells of tumor cells, etc. In some preferred embodiments, the antigen-presenting cell is a dendritic cell.

The term "treatment" used herein means that after afflicting from a disease, a subject is brought into contact (*e*.*g*. administration) with the immune adjuvant of the present disclosure or a pharmaceutical composition containing the same (hereinafter referred to as "pharmaceutical composition" of the present disclosure), thereby completely or partially easing, ameliorating, alleviating, suppressing symptoms of the disease, delaying onset of the disease, reducing the severity and/or reducing the incidence of one or more symptoms or characteristics of a particular disease, disorder and/or condition as compared to the case of no contact, which does not mean that the symptoms of the disease have to be completely suppressed. Affliction from a disease refers to occurrence of symptoms of the disease in the body.

The term "prevention" used herein means that before afflicting from a disease, a subject is brought into contact (*e.g.* administration) with the pharmaceutical composition of the present disclosure or the like, thereby alleviating symptoms after affliction from the disease as compared to the case of no contact, which does not mean that illness has to be completely suppressed.

The term "subject" used herein includes mammals. Mammals include, but are not limited to, domesticated animals (*e.g.* cattle, sheep, cats, dogs, and horses), primates (*e.g.* humans and non-human primates such as monkeys), rabbits, and rodents (*e.g.* mice and rats). In some embodiments, the individual or subject is a human.

The term "therapeutically effective amount" used herein refers to an amount effective to achieve the desired therapeutic result at a desired dose and for a given period of time. The therapeutically effective amount of an immune adjuvant or a pharmaceutical composition may vary depending on many factors such as the disease state, the age, sex and weight of the individual, and the ability of the immune adjuvant or pharmaceutical composition to elicit the desired response in the individual.

The terms "tumor" and "cancer" are used herein interchangeably and encompass solid tumors and liquid tumors.

### Immune Adjuvant

The immune adjuvant provided in the present disclosure is an erythrocyte-derived vesicle, and the vesicle comprises an erythrocyte content and a biomembrane structure encapsulating the erythrocyte content.

The immune adjuvant can promote generation of immune responses and is an immunopotentiator. In some embodiments, the immune adjuvant specifically augments the acquired immune response. Substances that promote an immune response other than antigens, antigen-presenting cells, T cells, and B cells may all be referred to as immune adjuvants in a broad sense.

There are two major types of immune adjuvants: one is antigens modified to promote the uptake and presentation of the antigens by antigen-presenting cells (including associated artificial carriers, modifications of antigens, etc.); and the other is exogenous activators (*e.g.* CpG-ODN, Poly I:C, and LPS) that activate the antigen-presenting cells (*e.g.* dendritic cells). It is unclear how the body exerts its immune surveillance function through endogenous regulation, and whether endogenous substances can effectively activate the acquired immune response.

The present disclosure has discovered through researches that after being swallowed by dendritic cells, the erythrocyte-derived vesicles can activate the dendritic cells and induce the expression of costimulatory molecules to induce specific T cellular immune responses and promote the release of cytokines, and exert better anti-tumor and anti-infection immunotherapeutic effects. The erythrocyte-derived vesicle takes the erythrocyte content and the biomembrane structure encapsulating the erythrocyte content as a whole, and their interconnection and cooperation enable the erythrocyte-derived vesicle as the body's endogenous substance to activate the body's immune system and effectively augment cellular immune responses. Moreover, this process does not cause toxic and side effects to the body, and the problem about immune toxicity caused by other immune adjuvants is effectively resolved. Besides, erythrocytes are numerous in the body and are accessible. A sufficient amount of erythrocyte-derived vesicles can be acquired through simple induction, which effectively overcome the problems about complex preparation steps and high cost existing in currently available immune adjuvants.

In some embodiments, erythrocyte-derived vesicles are released by physiologically non-induced erythrocytes. In some embodiments, erythrocyte-derived vesicles are released by apoptosis-induced erythrocytes. Extracellular vesicles released by either non-induced or apoptosis-induced erythrocytes can all effectively activate antigen-presenting cells, in particular dendritic cells, and promote the expression of costimulatory molecules and release of cytokines, thereby effectively enhancing the cellular immune response course of the body and exerting the immunotherapeutic effects against tumors, microbial infections, etc. Furthermore, by inducing apoptosis in erythrocytes, the number of vesicles released extracellularly by erythrocytes can be increased.

In some embodiments, the erythrocyte-derived vesicle has a particle size of 50 to 500 nm. In some preferred embodiments, the erythrocyte-derived vesicle has a particle size of 100 to 500 nm. Exemplarily, the erythrocyte-derived vesicle has a particle size of 50 nm, 80 nm, 100 nm, 150 nm, 180 nm, 200 nm, 250 nm, 280 nm, 300 nm, 350 nm, 400 nm, 450 nm, etc. In some more preferred embodiments, the erythrocyte-derived vesicle has a particle size of about 180 nm.

Further, the erythrocyte-derived vesicle may be mixed with one or more pharmaceutically acceptable carriers and prepared into any clinically available dosage form. In the present disclosure, the term "pharmaceutically acceptable carrier" refers to excipient ingredients that are compatible with cells, tissues or organs of the human or animal body and do not cause toxic and side effects such as toxicity, irritation, and allergy. Exemplarily, pharmaceutically acceptable carriers are known in the art, including at least one of a solvent, a solubilizer, a cosolvent, an emulsifier, a corrigent, an odor-masking agent, a colorant, a binder, a disintegrant, a filler, a lubricant, a wetting agent, an osmotic pressure regulator, a pH regulator, a stabilizer, a surfactant, and a preservative.

In some embodiments, the dosage form for administration of the immune adjuvant may be a liquid dosage form, a solid dosage form or a semi-solid dosage form. In some embodiments, the liquid dosage form may be solutions (including true solutions and colloidal solutions), emulsions (including O/W type, W/O type, and multiple emulsions), suspensions, injections (including aqueous injections, powder injections, and infusion solutions), eye drops, nasal drops, lotions or liniments or the like. In some embodiments, the solid dosage form may be tablets (including ordinary tablets, enteric-coated tablets, lozenges, dispersible tablets, chewable tablets, effervescent tablets, orally disintegrating tablets), capsules (including hard capsules, soft capsules, enteric-coated capsules), granules, powders, pellets, dropping pills, suppositories, films, patches, aerosols (dry powder inhalations), sprays, etc. In some embodiments, the semi-solid dosage form may be ointments, gels, pastes, etc. In some preferred embodiments, the immune adjuvant is prepared into an injection.

In order to make the immune adjuvant into an injection, water, ethanol, isopropanol, propylene glycol or a mixture thereof may be used as the solvent and appropriate amounts of solubilizer, cosolvent, pH regulator, and osmotic pressure regulator commonly used in this field may be added. The solubilizer or cosolvent may be poloxamer, lecithin, hydroxypropyl-β-cyclodextrin, etc. The pH regulator may be phosphate, acetate, hydrochloric acid, sodium hydroxide, etc. The osmotic pressure regulator may be sodium chloride, mannitol, glucose, phosphate, acetate, etc. If lyophilized powder is prepared, mannitol, glucose or the like may be further added as a supporting agent.

In order to make the immune adjuvant into a capsule, a therapeutically effective amount of erythrocyte-derived vesicles may be mixed with a diluent and a glidant, and the mixture may be put directly into a hard or soft capsule. Alternatively, a therapeutically effective amount of erythrocyte-derived vesicles may be first mixed with a diluent, a binder, and a disintegrant to be prepared into granules or pellets, and then put in a hard capsule or soft capsule. Various types of diluents, binders, wetting agents, disintegrants, and glidants used to prepare erythrocyte-derived vesicles may also be used to prepare capsules of erythrocyte-derived vesicles.

### Composition

The composition provided in the present disclosure comprises the immune adjuvant described above, which may effectively activate dendritic lymphocytes, promote the release of costimulatory factors and cytokines, augment the acquired cellular immune response, and has both safety and effectiveness.

In order to further improve the immunotherapeutic effect, the composition further comprises an immunotherapeutic agent. In some embodiments, the immunotherapeutic agent is an antigen, an antigen-presenting cell, an immunomodulator or any other reagents capable of promoting or mediating an immune response. In some embodiments, the immunotherapeutic agent may comprise an immunomodulator. The "immunomodulator" comprises an immune checkpoint modulator, for example, an immune checkpoint protein receptor and its ligand mediates inhibition of T cell-mediated cytotoxicity, and is typically expressed by a tumor cell or on a non-reactive T cell in a tumor microenvironment and allows the tumor to evade immune attack. An activity inhibitor of the immunosuppressive checkpoint protein receptor and its ligand may overcome the immunosuppressive tumor environment to allow the tumor to be attacked by its cytotoxic T cells. Examples of the immune checkpoint protein include, but are not limited to, PD-1, PD-L1, PDL2, CTLA4, LAG3, TIM3, TIGIT, and CD103. Modulation (including inhibition) of activities of such proteins may be accomplished by immune checkpoint modulators, which may include, for example, antibodies, aptamers, small molecules, and soluble forms of checkpoint receptor proteins that target checkpoint proteins. In addition, the "immunomodulator" further includes cytokines such as interleukin (IL), interferon (IFN), tumor necrosis factor (TNF), granulocyte-macrophage colony-stimulating factor (GM-CSF) and macrophage colony-stimulating factor (M-CSF), all of which can achieve the effect of augmenting the body's immune response and immune regulation of tumor subjects or pathogen-infected subjects.

In some embodiments, the immune adjuvant is combined with an antigen to produce a composition. Furthermore, the antigen is a tumor antigen or a microbial antigen. By combining the antigen with the immune adjuvant, it is possible to achieve good effects of preventing or treating tumors or resisting infections.

In some embodiments, the immune adjuvant is independent of the degree of binding to the antigen, and the immune adjuvant and the antigen can achieve a better immunotherapeutic effect only by means of simple physical mixing without a need to bind them chemically.

In some embodiments, the immune adjuvant is combined with an antigen-presenting cell to produce a composition. In some preferred embodiments, the immune adjuvant is combined with a dendritic cell to produce a composition. By combining the immune adjuvant with the dendritic cell, a good anti-tumor or anti-infectious effect may be achieved in the absence of an antigen.

### Use of Immune Adjuvant or Composition

The immune adjuvant or composition of the present disclosure may be used in at least one of (a) to (c) as follows:
(a) preparing a medicament for inducing an immune response or activating an antigen-presenting cell;
(b) preparing a medicament for preventing or treating a tumor; and
(c) preparing a medicament for preventing or treating a microbial infection.

The medicament for inducing an immune response or activating an antigen-presenting cell that is prepared by the immune adjuvant or the composition can effectively augment the body's acquired immune response, reduce immune escape, and exert a good anti-tumor or anti-infectious effect.

In some preferred embodiments, the antigen-presenting cell is a dendritic cell.

In some preferred embodiments, the immune adjuvant acts as the sole active ingredient in the medicament that activates antigen-presenting cells. Erythrocyte-derived vesicles are not limited to the degree of binding to antigens, that is, they may effectively activate dendritic cells, target the majority of tumors and infections, and have good universality.

### Preparation Method for Immune Adjuvant

The present disclosure provides a method for preparing an immune adjuvant, comprising the following steps:
culturing step: culturing an erythrocyte that secretes a vesicle into a culture environment; and
collection step: collecting the vesicle in the culture environment, which is the immune adjuvant.

In some embodiments, the culturing step is to put erythrocytes in a medium so that the erythrocytes are cultured in a natural environment suitable for their growth and metabolism, and the naturally cultured erythrocytes secrete vesicles into the medium. In some embodiments, the culturing step further comprises subjecting the erythrocytes to apoptosis-inducing treatment, such that the apoptosis-induced erythrocytes secrete vesicles into the culture environment. Exemplarily, the apoptosis-inducing treatment of erythrocytes may be irradiation of the erythrocytes with ultraviolet light or other treatment methods used in the art to induce apoptosis.

In some embodiments, the collection step is to centrifuge the culture solution containing erythrocytes by the density gradient centrifugation method, and collect the centrifuged vesicles, which are immune adjuvants. Preferably, the density gradient centrifugation is conducted under the condition of 1000 to 50000 g.

The preparation method for the immune adjuvant provided in the present disclosure is simple in steps and easy to operate, and the extracted vesicles have a homogeneous particle size and good morphology and thus have the conditions for clinical application.

### Examples

The embodiments of the present disclosure will be described in detail below with reference to the examples. However, a person skilled in the art will appreciate that the following examples are only intended to illustrate the present disclosure and should not be construed as limiting the scope of the present disclosure. Where specific conditions are not specified in the examples, the conventional conditions or the conditions recommended by the manufacturers shall be followed. Reagents or instruments used here, for which the manufacturers are not indicated, are all commercially-available conventional products.

The following are various cells, medicaments, experimental animals, instruments and equipment, and formulation of some solutions used in the Examples:
Antibody: mouse OVA^{257-264H-2kb} tetramer antibody purchased from HELIXGEN (Guangzhou) Co., Ltd., Cat. No.: HG08T14028.
Cells: H22 mouse hepatoma cells (H22 cells), Lewis mouse lung carcinoma cells, and B16 mouse melanoma cells, all of which may be purchased from ATCC (U.S.A.) or from China Center for Type Culture Collection CCTCC.
Test animals: BALB/c mice and C57/bl mice purchased from Hubei Medical Experimental Research Center affiliated with Hubei Provincial Center for Disease Control and Prevention.
Dendritic cells are acquired by isolating bone marrow cells from mice and inducing them by IL-4 and GM-CSF for 5 days.
Instruments and equipment: HITACHI HT7700 transmission electron microscope, Malvern Nanosight NS300 nanoparticle tracker, and BD acurri C6 type flow cytometer.
The amino acid sequence of ovalbumin polypeptide (OVA²⁵⁷⁻²⁶⁴) used in the following Examples is the sequence set forth in SEQ ID NO: 1.

### Example 1: Particle size determination and electron microscopy observation of cell vesicles produced by ultraviolet light-induced apoptosis in mouse erythrocytes

### 1. Experimental Materials and Reagents

Wild type C57/bl mice, erythrocytes from eyeball blood

### 2. Experimental Steps

(1) Eyeball blood of C57/bl mice was collected and centrifuged at 2000 rpm for 5 min, and the supernatant was removed. The erythrocytes were washed three times with a Hank's solution free of Ca²⁺ and Mg²⁺, with the first two times centrifuged at 2000 rpm for 5 min and the last time centrifuged at 2000 rpm for 10 min, and the supernatant was removed. A hemocytometer was used to calculate the erythrocyte concentration, and the erythrocytes accumulated in the lower layer were formulated into the corresponding concentration, from which 3×10⁸ of mouse erythrocytes were measured.
   The above cells were transferred to 10 ml of 1640 cell culture media respectively, then irradiated with UV for 1 h, and incubated for 20 h.
(2) The resulting culture media containing mouse erythrocyte-derived microparticles were centrifuged stepwise, and the supernatant was collected. That is, each of the culture media containing mouse erythrocyte-derived microparticles was centrifuged for 10 min at a rotational speed of 2000 rpm to remove cells and fragments.
   The supernatant obtained above was centrifuged at a centrifugal force of 14000 g for 1.5 min. The centrifuged supernatant was collected and further centrifuged at a centrifugal force of 14000 g for 1 h. The resulting precipitates were re-suspended with 1 ml of PBS respectively to obtain mouse erythrocyte-derived microparticles. Thereafter, the microparticles were re-suspended and washed with PBS two times for subsequent experiments.
(3) After the resulting mouse erythrocyte-derived microparticles were washed, their particle sizes were detected by a nanoparticle tracker, or after electron microscope sample pretreatment, the samples were observed under a scanning electron microscope and a transmission electron microscope (the experimental steps were the same as those by conventional electron microscopy observation); afterwards, the samples were observed under an electron microscope, and an appropriate field of view was selected for photographing and recording.

### 3. Experimental Results

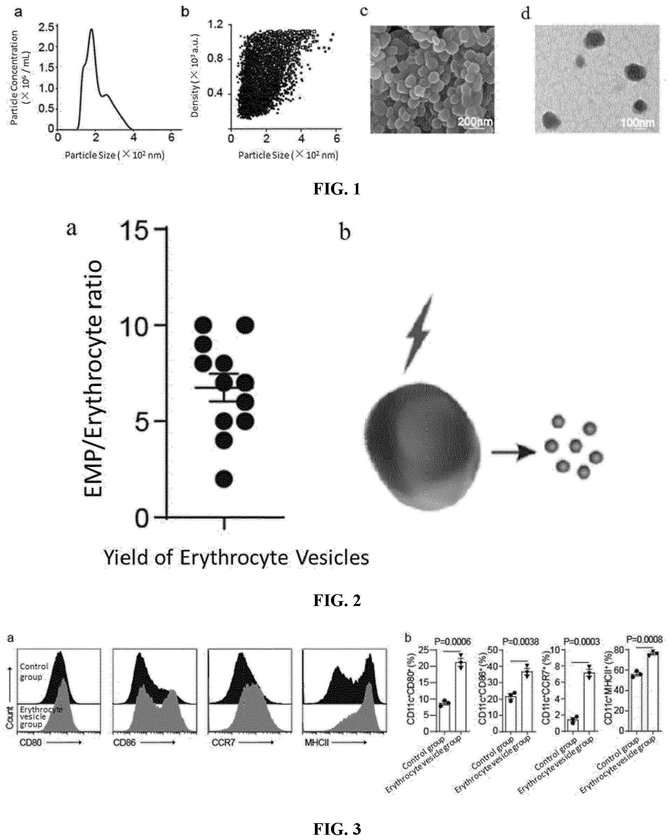

FIG. 1 showed the results of the particle size and morphology of erythrocyte-derived vesicles. FIG. 1a and FIG. 1b showed the particle size of the erythrocyte vesicles detected by a nanoparticle tracker (FIG. 1a showed the particle concentration of the erythrocyte vesicles with different particle sizes, and FIG. 1b showed the particle density of the erythrocyte vesicles with different particle sizes). As could be appreciated from FIG. 1a and FIG. 1d, the particle size distribution of the erythrocyte-derived vesicles was relatively uniform, concentrated at about 180 nm. FIG. 1c and FIG. 1d showed the morphology of the erythrocyte-derived vesicles observed by scanning electron microscope and transmission electron microscope, respectively. As could be appreciated from FIG. 1c and FIG. 1d, the morphology of the erythrocyte vesicles was homogeneous, and their particle size distribution was consistent with the detection result by the nanoparticle tracker.

### Example 2: Detection of yield of microparticles released after induction of apoptosis in mouse erythrocytes

### 1. Experimental Materials and Reagents

Mouse erythrocytes, a nanoparticle tracker.

### 2. Experimental Steps

(1) According to the method of Example 1, blood was collected from mice, erythrocytes were isolated, and the erythrocyte concentration was calculated. 1×10⁸ mouse erythrocytes were taken therefrom.
(2) Mouse erythrocyte-derived microparticles were prepared and extracted according to the method of Example 1.
(3) 1 ml of the mouse erythrocyte-derived microparticles obtained above was centrifuged at a centrifugal force of 14000 g for 1 h. The supernatant was removed. The resulting precipitate was washed and re-suspended with 500 µL of PBS, which was repeated twice.
(4) 500 µL of the solution containing the mouse erythrocyte-derived microparticles obtained above were detected with a nanoparticle tracker to determine its concentration.

### 3. Experimental Results

FIG. 2 showed the results of the yield of erythrocyte-derived vesicles. Specifically, FIG. 2a was the yield result of erythrocyte vesicles, and FIG. 2b was a schematic diagram for production of vesicles by erythrocytes. By measuring and calculating the concentrations of erythrocyte vesicles in multiple batches, it was determined that in case of apoptosis, each erythrocyte could induce the production of approximate seven erythrocyte microparticles on average.

### Example 3: Influence of erythrocyte microparticles on costimulatory molecules of dendritic cells

### 1. Experimental Materials and Reagents

C57/bl female mice aged 6 to 8 weeks, recombinant mouse interleukin-4 (IL-4), and recombinant mouse granulocyte-macrophage colony-stimulating factor (GM-CSF).

### 2. Experimental Steps

(1) Bone marrow tissues were collected from the fibula-tibia of C57/bl female mice, ground, and pipetted up-down, and then the erythrocytes were removed using an erythrocyte lysis solution. The resulting product was washed twice with complete medium (RPMI1640 medium containing 10% fetal bovine serum), and inoculated into a low-adhesion culture plate (1×10⁶/per well/3 ml). Murine IL-4 (20 ng/ml) and GM-CSF (20 ng/ml) were added. The medium was changed every other day. They were cultured till day 6 to obtain mature dendritic cells.
(2) Erythrocyte microparticles were obtained by the same step described previously, and co-incubated with the mature dendritic cells at a ratio of 1: 100 (cells: microparticles = 1: 100). After 48 h, the dendritic cells were collected. The expression of costimulatory molecules of the dendritic cells were detected by the flow cytometry, including CD80, CD86, CCR7, and MHCII. The results were shown in FIG. 3. FIG. 3a showed a flow chart of CD80, CD86, CCR7, and MHCII detected by the flow cytometry, and FIG. 3b was the quantization result of the expression of costimulatory molecules shown in FIG. 3a.

### 3. Experimental Results

As could be seen from FIG. 3, the erythrocyte microparticles significantly promoted the expression of various kinds of activation molecules in the dendritic cells.

### Example 4: Influence of erythrocyte microparticles on expression of cytokines in dendritic cells

### 1. Experimental Materials and Reagents

C57/bl female mice aged 6 to 8 weeks, recombinant mouse interleukin-4 (IL-4), recombinant mouse granulocyte-macrophage colony-stimulating factor (GM-CSF), PCR primers for various kinds of cytokines, and ELISA kits (commercially available) for various kinds of cytokines.

### 2. Experimental Steps

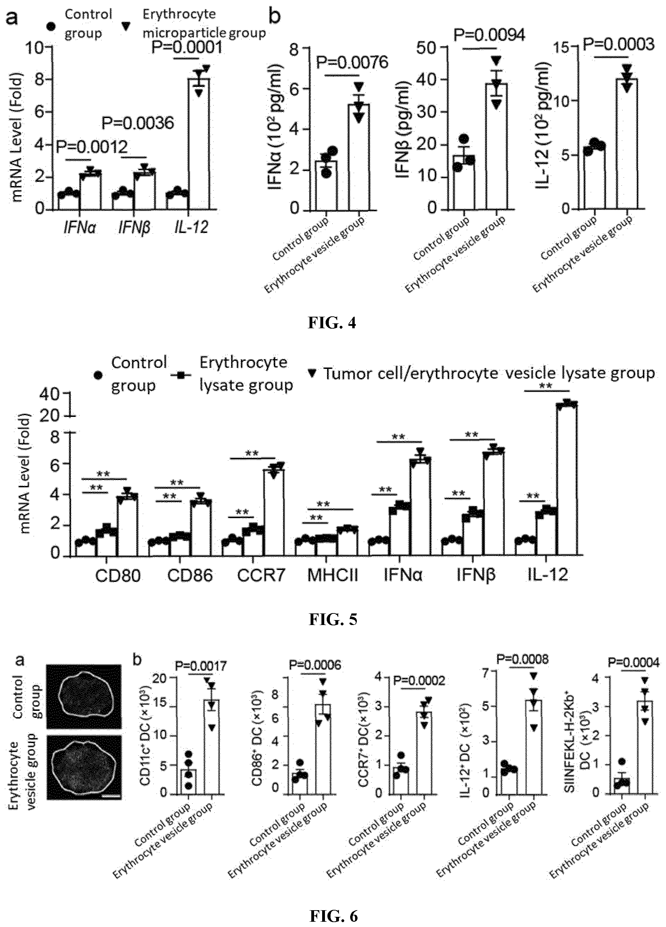

(1) Bone marrow tissues were collected from the fibula-tibia of C57/bl female mice, ground, and pipetted up-down, and then the erythrocytes were removed using an erythrocyte lysis solution. The resulting product was washed twice with complete medium (RPMI1640 medium containing 10% fetal bovine serum), and inoculated into a low-adhesion culture plate (1×10⁶/per well/3 ml). Murine IL-4 (20 ng/ml) and GM-CSF (20 ng/ml) were added. The medium was changed every other day. They were cultured till day 6 to obtain mature dendritic cells.
(2) Erythrocyte microparticles were obtained by the same step described previously, and co-incubated with the mature dendritic cells at a ratio of 1: 100 (cells: microparticles = 1: 100). After 12 h, the dendritic cells were collected, and RNAs were extracted from the cells for quantitative detection of the expression of IFN-α, IFN-β, and IL-12 by real-time quantitative PCR. The remaining wells were inoculated for 72 h. The culture supernatant was collected for quantitative detection of proteins (ELISA) of various kinds of cytokines. The results were shown in FIG. 4. Specifically, FIG. 4a showed the detection results of the mRNA expression of IFN-α, IFN-β, and IL-12, and FIG. 4b showed the detection results of the protein expression of IFN-α, IFN-β, and IL-12.

### 3. Experimental Results

As could be seen from FIG. 4, the erythrocyte microparticles significantly promoted the expression of various kinds of cytokines in the dendritic cells.

### Example 5: Influence of erythrocyte-derived microparticles and tumor lysates on dendritic cells

As in Example 4, B16 tumor cell lysates (2 ml of lysates were obtained from every 10⁶ cells, and 50 µL of the lysates were added to each well), erythrocyte microparticles, or a mixture of both were added to the dendritic cells. After 2 h, the dendritic cells were collected and RNAs were extracted from the cells and subjected to real time PCR for quantitative detection of the expression of CD80, CD86, MHCII, CCR7, IFN-α, IFN-β, and IL-12. The results were shown in FIG. 5, showing that the tumor cell lysates could slightly increase the expression of the above genes, while they greatly increased the expression of these dendritic cell activation marker genes in the presence of erythrocyte vesicles.

### Example 6: In vivo experiment to observe influence of erythrocyte vesicles on body's dendritic cells

### 1. Experimental Materials and Reagents

Mouse erythrocytes, C57/bl mice, OVA-B16 cells, relevant antibodies for flow cytometry.

### 2. Experimental Steps

(1) According to the method of Example 1, mouse erythrocyte-derived microparticles were prepared, isolated and extracted.
(2) The ovalbumin-expressing mouse melanoma cells (OVA-B16 cells) were frozen and thawed three times and the lysates were then collected (1 ml of lysates was collected from every 10⁷ cells). Afterwards, 20 µl of the lysates were measured and injected into the footpads of C57/bl mice, which was the control group. The test group was a mixture of tumor cell lysates and erythrocyte vesicles.
(3) After 24 h following the footpad injection in step (2), the mice were killed, the popliteal lymph nodes were isolated, and frozen sections were prepared for CD11c cell staining.
(4) On day 5 after the same treatment as in (2) was conducted, the lymph nodes were isolated, and then a single lymph node cell was collected for flow cytometry staining.

### 3. Experimental Results

FIG. 6 showed the activation of body's dendritic cells by erythrocyte-derived vesicles *in vivo.* Specifically, FIG. 6a was the fluorescence staining of frozen sections, showing that in the presence of erythrocyte vesicles, the OVA-B16 cell lysates could have an immunochemotactic response better, and more CD11c-positive dendritic cells were aggregated in lymph nodes.

After further detection by flow cytometry, the statistics showed that all of CD11c-positive, CCR7-positive, CD86-positive, IL-12-positive, and OVA^{257-264H-2Kb}-positive (SIINFEKL-H-2Kb⁺) dendritic cells increased significantly in the erythrocyte vesicle group (FIG. 6b), which had a definite statistical significance.

### Example 7: Comparison between activation situations of dendritic cells by in vivo erythrocyte vesicles and by other adjuvants

### 1. Experimental Materials and Reagents

C57/bl mice, MF59, aluminum adjuvant.

### 2. Experimental Steps

(1) According to the method of Example 1, mouse erythrocyte-derived microparticles were prepared, isolated and extracted.
(2) As did in Example 6, the OVA-B16 cell lysates were mixed with erythrocyte vesicles, MF59 or an aluminum adjuvant and injected into the footpads of C57/bl mice in different groups, separately.
(3) After 24 h following the footpad injection in step (2), the mice were killed, the popliteal lymph nodes were isolated, and the number of dendritic cells was analyzed by CD11c flow cytometry staining.
(4) After 5 days following the footpad injection in step (2), the mice were killed, the popliteal lymph nodes were isolated, and the maturation of the dendritic cells was analyzed by flow cytometry staining, including the proportions of CD11c-positive, CCR7-positive, CD86-positive, IL-12-positive, and OVA^{257-264H-2Kb}-positive cells.

### 3. Experimental Results

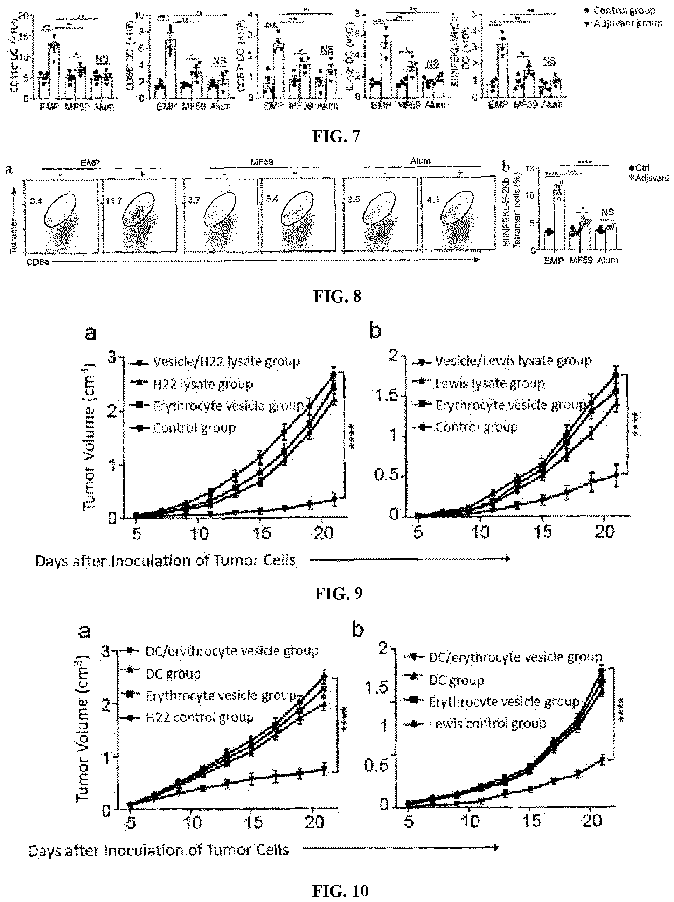

FIG. 7 showed the result that compared with adjuvants MF59 and aluminum commonly used in clinical practice, the erythrocyte vesicles could have a better effect of activating the immune system and allow the dendritic cells to be recruited to the draining lymph nodes.

FIG. 7 further showed the result that as an immune adjuvant, the erythrocyte vesicles could play a better role in making the dendritic cells express CD86, CCR7, and MHCII costimulatory molecules and could also promote secretion of IL-12, and the expression of OVA^{257-264H-2Kb} conjugate on the membrane surface was also significantly increased during the antigen presentation.

### Example 8: Comparison between activation situations of body's specific T cells by in vivo erythrocyte vesicles and by other adjuvants

### 1. Experimental Materials and Reagents

C57/bl mice, mouse OVA^{257-264H-2kb} tetramer antibody.

### 2. Experimental Steps

(1) According to the method of Example 1, mouse erythrocyte-derived microparticles were prepared, isolated and extracted.
(2) As did in Example 6, the OVA-B16 cell lysates were mixed with erythrocyte vesicles, MF59 or an aluminum adjuvant and injected into the footpads of C57/bl mice in different groups, separately.
(3) After 5 days following the footpad injection in step (2), the mice were killed, the popliteal lymph nodes were isolated, and the number of specific T cells was analyzed by flow cytometry staining.

### 3. Experimental Results

The results were shown in FIG. 8. Specifically, FIG. 8a showed the detection results of CD8a-positive cells in mice treated with erythrocyte microparticles (EMPs), MF59, and aluminum (Alum); and FIG. 8b showed the detection results of OVA^{257-264H-2Kb}-positive cells in mice treated with EMP, MF59, and Alum. As was clear from FIG. 8, the erythrocyte-derived vesicles could act effectively as an adjuvant to induce production of OVA^{257-264H-2kb}-specific T cells, and the effect was significantly better than those achieved by MF-59 and aluminum adjuvant.

### Example 9: Prophylactic and therapeutic effects of erythrocyte vesicles as an immune adjuvant on mouse tumor

### 1. Experimental Materials

H22 mouse hepatoma cells, Lewis mouse lung carcinoma cells, BALB/c mice and C57/bl mice, 10 mice for each experiment.

### 2. Experimental Steps

2.1
   (1) The tumor inoculation day was determined as day 0. PBS, tumor cell lysates, erythrocyte vesicles, or a mixture of the tumor cell lysates and erythrocyte vesicles were subcutaneously vaccinated on day -14, day -13, and day -7, respectively (the total volume was 50 µL/mouse; the tumor cell lysates were prepared as described previously; the number of erythrocyte vesicles in the groups was 2.5×10⁸; NTA counting was adopted). On day 0, the corresponding tumor cells were subcutaneously inoculated (H22 cells inoculation group was for BALB/c mice, and Lewis cells inoculation group was for C57/bl mice). Each mouse was inoculated with 3×10⁵ tumor cells in a volume of 50 µL.
   (2) The subcutaneous tumors of mice were observed and the volumes were measured.
2.2
   (1) BALB/c mice and C57/bl mice were inoculated with H22 mouse hepatoma cells and Lewis mouse lung carcinoma cells, respectively. When the tumors grew to a volume of 5×5 mm³, mouse bone marrow-derived dendritic cells DCs (cultured by the same method described previously) were injected into the tail vein. Each mouse was injected with 1×10⁶ cells each time, and injected once every five days, three times in total. The erythrocyte vesicle-induced dendritic cells were obtained by the same method as described previously.
   (2) The subcutaneous tumors of mice were observed and the volumes were measured.

### 3. Experimental Results

FIG. 9 showed the prophylactic effect of erythrocyte-derived vesicles as an immune adjuvant on the tumor. Specifically, FIG. 9a showed the prophylactic effects on the tumor in the vesicle/H22 lysate group, H22 lysate group, erythrocyte vesicle group, and PBS control group; and FIG. 9b showed the prophylactic effects on the tumor in the vesicle/Lewis lysate group, Lewis lysate group, erythrocyte vesicle group, and PBS control group. By measuring the tumor volumes, it was found that the tumor growth was significantly inhibited in the group inoculated with a mixture of erythrocyte-derived vesicles and tumor cell lysates, while the tumor inhibitory effect was not found in the group inoculated with erythrocyte-derived vesicles or tumor cell lysates alone. These findings indicated that the erythrocyte-derived vesicles could exert a good anti-tumor effect in the presence of a tumor antigen. Moreover, without a need to be fused, erythrocyte-derived vesicles and tumor antigens could play a significant anti-tumor effect only by simply mixing them.

FIG. 10 showed the therapeutic effect of the erythrocyte-derived vesicles as an immune adjuvant in combination with the dendritic cells on the tumor. Specifically, FIG. 10a showed the therapeutic effects on the tumor in the DC/erythrocyte vesicle group, DC group, erythrocyte vesicle group, and H22 control group; and FIG. 10b showed the therapeutic effects on the tumor in the DC/erythrocyte vesicle group, DC group, erythrocyte vesicle group, and Lewis control group. By measuring the tumor volumes, it was found that reinfusion of dendritic cells alone could only slightly hinder the tumor growth, and injection of EMP alone could not effectively inhibit the tumor growth in mice, either. However, after reinfusion of dendritic cells treated with EMP, the tumor growth could be effectively inhibited. These findings indicated that EMP could act effectively as an immune adjuvant to promote the anti-tumor function of the dendritic cells *in vivo.*

### Example 10: Prophylactic and therapeutic effects of erythrocyte vesicles as an immune adjuvant on Listeria monocytogenes

### 1. Experimental Materials

Erythrocyte vesicles, C57/bl mice, ovalbumin-expressing recombinant *Listeria monocytogenes* (OVA*-Listeria monocytogenes*), ovalbumin (OVA), ovalbumin polypeptide (OVA²⁵⁷⁻²⁶⁴, SIINFEKL), LB medium

### 2. Experimental Steps

2.1
(1) 24 h before inoculation with OVA*-Listeria monocytogenes,* OVA and OVA²¹⁷⁻²⁶⁴ were prophylactically inoculated into the tail vein, or the protein and polypeptide were mixed with the erythrocyte vesicles and then prophylactically inoculated. Saline was injected as a blank control.
(2) 24 h later, each mouse was inoculated with 1×10⁵ OVA*-Listeria monocytogenes* into the tail vein. After 24 h following the bacterial inoculation, the mice were dissected. The livers and spleens in mice were harvested and ground. Thereafter, the ground tissue fluid was measured and inoculated on an LB medium plate. After 16 h, the colony forming units on the plate was observed and recorded.

2.2
(1) Each mouse was inoculated with 1×10⁵ OVA*-Listeria monocytogenes* into the tail vein.
(2) After 24 h following the bacterial inoculation in step (1), the dendritic cells treated in different ways were injected into the tail vein, including OVA, OVA²⁵⁷⁻²⁶⁴, and their respective mixtures with the erythrocyte vesicles to induce the dendritic cells. Untreated dendritic cells were used as a control (1 × 10⁶ dendritic cells were injected in each group), and then the survival of the mice was observed.

### 3. Experimental Results

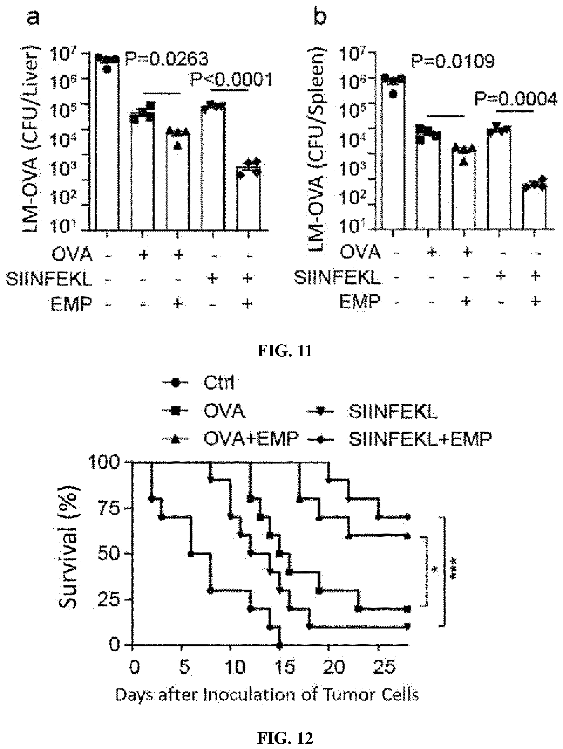

FIG. 11 showed the prophylactic effect of the erythrocyte-derived vesicles as an immune adjuvant on the *Listeria monocytogenes* infection. FIG. 11a showed the prophylactic effect of the erythrocyte-derived vesicles as an immune adjuvant on the *Listeria monocytogenes* infection in mouse liver; and FIG. 11b showed the prophylactic effect of the erythrocyte-derived vesicles as an immune adjuvant on the *Listeria monocytogenes* infection in mouse spleen. By counting the colony forming units on the plates, it was clearly found that the erythrocyte-derived vesicles, when co-inoculated with the OVA protein or OVA²⁵⁷⁻²⁶⁴, could significantly inhibit the growth of OVA-*Listeria monocytogenes* (the colony forming units in the liver and spleen were significantly reduced). The present example indicated that in prophylactic vaccinations, the erythrocyte-derived vesicles could effectively improve the antibacterial infection effect, suggesting that it could be used as an immune adjuvant to improve the protective effect of vaccines in the course of bacterial infections.

FIG. 12 showed the therapeutic effect of the erythrocyte-derived vesicles in combination with the dendritic cells on the *Listeria monocytogenes* infection. Through the observation of the survival time, it could be found that compared with the dendritic cells induced by the OVA protein or OVA polypeptide alone, the dendritic cells induced jointly by the erythrocyte microparticles (EMPs) and the OVA protein or OVA polypeptide could be better in producing an acquired immune response (better clearance of the OVA *Listeria monocytogenes*), and the mice infected with bacteria could survive longer. These findings indicated that EMP could act as a good immune adjuvant to enhance the therapeutic effects of dendritic cell-based therapeutic vaccines in the course of bacterial infections.

### Example 11: Safety of inoculation of erythrocyte vesicles

### 1. Experimental Materials

Erythrocyte vesicles, C57/bl mice

### 2. Experimental Steps

C57/bl mice were subcutaneously inoculated with EMP or a mixture of EMP and OVA proteins (in the same quantities as described previously) and injected once a day for 3 consecutive days. The activity statuses of the mice were then observed and their body weight was weighed.

### 3. Experimental Results

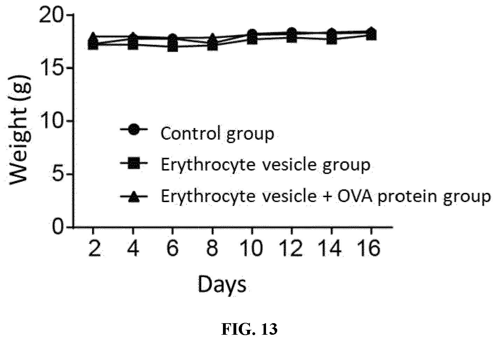

Regardless of the mice being inoculated with EMPs alone or with a mixture of EMP and OVA proteins, the body weight of mice was not affected, and there was no significant difference in the daily activities of the mice as compared to the control group (FIG. 13). This example indicated that as an immune adjuvant, EMP did not cause obvious toxic and side effects.

The above examples of the present disclosure are provided only for clear illustration of the present disclosure, instead of limiting the embodiments provided herein. For those of ordinary skill in the art, other different forms of variations or alternations may be further made on the basis of the above illustration. Here it is not necessary to exhaust all embodiments, nor is it possible. Any modifications, equivalent substitutions, improvements, etc. made within the spirit and principles of the present disclosure shall be encompassed within the scope of protection for the claims of the present disclosure.

## Claims

1. An immune adjuvant, wherein the immune adjuvant is an erythrocyte-derived vesicle, and the vesicle comprises an erythrocyte content and a biomembrane structure encapsulating the erythrocyte content.

2. The immune adjuvant according to claim 1, wherein the vesicle is an extracellular vesicle released by an erythrocyte; optionally, the erythrocyte is a non-induced or apoptosis-induced erythrocyte.

3. The immune adjuvant according to claim 1 or 2, wherein the vesicle has a particle size of 50 to 500 nm; preferably, the vesicle has a particle size of 100 to 500 nm.

4. The immune adjuvant according to any one of claims 1 to 3, wherein the immune adjuvant further comprises one or more pharmaceutically acceptable carriers; optionally, the pharmaceutically acceptable carriers comprise one or a combination of two or more of a solvent, a solubilizer, a cosolvent, an emulsifier, a corrigent, an odor-masking agent, a colorant, a binder, a disintegrant, a filler, a lubricant, a wetting agent, an osmotic pressure regulator, a pH regulator, a stabilizer, a surfactant, and a preservative.

5. The immune adjuvant according to any one of claims 1 to 4, wherein the immune adjuvant is a tablet, a capsule, an injection, a spray, a granule, a pulvis, a suppository, a pill, a cream, a paste, a gel, a powder, an oral solution, an inhalant, a suspension or a dry suspension; preferably, the immune adjuvant is an injection.

6. A composition, comprising the immune adjuvant according to any one of claims 1 to 5.

7. The composition according to claim 6, wherein the composition further comprises an immunotherapeutic agent; preferably, the immunotherapeutic agent is at least one selected from the group consisting of an antigen, an antigen-presenting cell, and an immunomodulator; more preferably, the antigen-presenting cell is a dendritic cell.

8. Use of the immune adjuvant according to any one of claims 1 to 5 or the composition according to claim 6 or 7 in at least one of (a) to (c) as follows:
(a) preparing a medicament for inducing an immune response or activating an antigen-presenting cell;
(b) preparing a medicament for preventing or treating a tumor; and
(c) preparing a medicament for preventing or treating a microbial infection;
preferably, the antigen-presenting cell is a dendritic cell.

9. A method for preparing the immune adjuvant according to any one of claims 1 to 5, comprising the following steps:
culturing step: culturing an erythrocyte that secretes a vesicle into a culture environment; and
collection step: collecting the vesicle in the culture environment, which is the immune adjuvant;
optionally, the culturing step further comprises subjecting the erythrocyte to apoptosis-inducing treatment, such that an apoptosis-induced erythrocyte secretes the vesicle into the culture environment;
optionally, the apoptosis-inducing treatment comprises irradiating the erythrocyte with ultraviolet light.

10. The method according to claim 9, wherein the collection step comprises subjecting the erythrocyte in the culture environment to density gradient centrifugation, and collecting the vesicle after centrifugation; preferably, the density gradient centrifugation is carried out under the condition of 1000 to 50000 g.
